(19) Europäisches Patentamt
European Patent Office
Office européen des brevets

(11) **EP 4 005 473 A1**

(12) **EUROPEAN PATENT APPLICATION**
published in accordance with Art. 153(4) EPC

(43) Date of publication:
**01.06.2022 Bulletin 2022/22**

(51) International Patent Classification (IPC):
***A61B 5/11*** *(2006.01)*     ***A61F 2/72*** *(2006.01)*

(21) Application number: **20860220.1**

(22) Date of filing: **17.08.2020**

(86) International application number:
**PCT/CN2020/109444**

(87) International publication number:
**WO 2021/042970 (11.03.2021 Gazette 2021/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.09.2019 CN 201910827590**

(71) Applicant: **Jingdong Technology Information Technology Co., Ltd.**
**Beijing 100176 (CN)**

(72) Inventors:
• **TIAN, Yanxiu**
 **Beijing 100176 (CN)**
• **HAN, Jiuqi**
 **Beijing 100176 (CN)**
• **NIU, Tianzeng**
 **Beijing 100176 (CN)**

(74) Representative: **Ruuskanen, Juha-Pekka et al**
**Page White & Farrer**
**Bedford House**
**John Street**
**London WC1N 2BF (GB)**

(54) **MOTION SPEED ANALYSIS METHOD AND APPARATUS, AND WEARABLE DEVICE**

(57)    A motion speed analysis method and apparatus, and a wearable device, the motion speed analysis method comprising: acquiring a surface electromyogram signal of a detected user (101); determining an action segment signal according to the surface electromyogram signal (102); determining the rate of change of the potential of the action segment signal (103); and determining the motion speed level according to the rate of change and a predetermined rate of change threshold (104). By means of the described method, the motion speed level may be determined according to a surface electromyogram signal generated by an organism adjusting muscle contraction in order to perform a movement operation, and motion speeds comprising amputees are analyzed, thereby expanding the application range of motion speed analysis.

EP 4 005 473 A1

acquire surface myoelectric signalS of a detected user — 101

↓

determine Signals of action segment according to the surface myoelectric signals — 102

↓

determine a change rate of potentials of the signals of action segment — 103

↓

determine a motion speed level according to the change rate and a preset change rate threshold — 104

Fig. 1

**Description**

**CROSS-REFERENCE TO RELATED APPLICATIONS**

This application is based on and claims the priority to the Chinese patent application No. 201910827590.8 filed on September 3, 2019, the disclosure of which is hereby incorporated in its entirety into the present application.

**TECHNICAL FIELD**

This disclosure relates to the technical field of intelligent wearable devices, and particularly, to a motion speed analysis method and apparatus, and wearable device.

**BACKGROUND**

A wearable intelligent device often needs to acquire a user's motion state, such as a gesture action, and may make different responses according to different motion velocities.
In the related art, an execution speed of the gesture action is calculated as follows:

(1) The execution speed of the gesture action is determined by using a conduction speed of action potentials of muscle fibers. A plurality of detection electrodes are arranged along directions of the muscle fibers, and the conduction speed is estimated by using a distance and transmission time of an electric signal between every two electrodes. A most direct method is to calculate the conduction speed according to the time and the transmission distance.
(2) An execution angular velocity of the action is measured with the aid of an external device.

**Summary**

According to an aspect of some embodiments of the present disclosure, there is provided a motion speed analysis method, comprising: acquiring surface myoelectric signals of a detected user; determining signals of action segment according to the surface myoelectric signals; determining a change rate of potentials of the signals of action segment; and determining a motion speed level according to the change rate and a preset change rate threshold
In some embodiments, determining the change rate of potentials of the signals of action segment comprises: determining a mean value of the potentials within a window from an initial position of the signals of action segment; sliding the window with a preset stride, and obtaining a mean value of the potentials within the window after each sliding; and taking a difference between mean values of the potentials of two windows spaced at a preset interval as a change rate of the potentials of a latter window of the two windows.
In some embodiments, determining the change rate of the potentials of the signals of action segment further comprises: comparing a mean value of the potentials of a current window with a preset first threshold; if the mean value of the potentials of the current window is greater than the preset first threshold, determining a difference between the mean value of the potentials of the current window and a mean value of the potentials of a former window with the preset interval from the current window, and taking the difference as a change rate of the potentials of the current window; and if the mean value of the potentials of the current window is less than or equal to the preset first threshold, ignoring the change rate of the potentials of the current window.
In some embodiments, determining the motion speed level according to the change rate and the preset change rate threshold comprises: comparing the change rate with the preset change rate threshold; if the change rate is less than or equal to the preset change rate threshold, determining that a motion state of the user is a first motion speed level; and if the change rate is greater than the preset change rate threshold, determining that the motion state of the user is a second motion speed level, wherein a speed at the second motion speed level is larger than the speed at the first motion speed level.
In some embodiments, determining the motion speed level according to the change rate and the preset change rate threshold comprises: determining a preset change rate threshold interval range where the change rate belongs to; and determining that at a position of a signal to which the change rate corresponds, a motion state of the user is a motion speed level to which the preset change rate threshold interval range corresponds, wherein the number of motion speed levels is greater than 2.
In some embodiments, the motion speed analysis method further comprises: respectively acquiring data of the surface myoelectric signals of the user in various motion speed states; and determining the preset change rate threshold according to the data of the surface myoelectric signals.
In some embodiments, acquiring the surface myoelectric signal of the detected user comprises: acquiring initial data of surface myoelectric signals; and acquiring the surface myoelectric signals by correcting the initial data of surface myoelectric signals based on a baseline threshold.

In some embodiments, acquiring the surface myoelectric signals of the detected user further comprises: determining the baseline threshold thr according to an equation:

$$\text{thr}=\text{mean}\{\text{MAV}_1,\text{MAV}_2,\text{MAV}_3,\ \ldots,\text{MAV}_k\}+A$$

wherein MAVi is a maximum value of the signals within a sliding window in resting-state data of the initial data of the surface myoelectric signals, i is a positive integer between 1 and k, k is the number of times that the sliding window slides, and A is a preset constant.

According to an aspect of other embodiments of the present disclosure, there is provided a motion speed analysis apparatus, comprising: a signal acquisition unit configured to acquire surface myoelectric signals of a detected user; an action segment determination unit configured to determine signals of action segment according to the surface myoelectric signals; a change rate determination unit configured to determine a change rate of potentials of the signals of action segment; and a speed level determination unit configured to determine a motion speed level according to the change rate and a preset change rate threshold.

In some embodiments, the change rate determination unit is configured to: determine a mean value of the potentials within a window from an initial position of the signals of action segment; slide the window with a preset stride, and obtaining a mean value of the potentials within the window after each sliding; and take a difference between mean values of the potentials of two windows spaced at a preset interval as a change rate of the potentials of a latter window of the two windows.

In some embodiments, the speed level determination unit is configured to: determine a preset change rate threshold interval range where the change rate belongs to; and determine that at a position of a signal to which the change rate corresponds, a motion state of the user is a motion speed level to which the preset change rate threshold interval range corresponds; wherein the number of motion speed levels is greater than 2.

According to an aspect of still other embodiments of the present disclosure, there is provided a motion speed analysis apparatus, comprising: a memory; and a processor coupled to the memory, wherein the processor is configured to, based on instructions stored in the memory, perform any of the above motion speed analysis methods.

According to an aspect of further embodiments of the present disclosure, there is provided a computer-readable storage medium having stored thereon computer program instructions, which when executed by a processor, implement steps of any of the above motion speed analysis methods.

Additionally, according to an aspect of some embodiments of the present disclosure, there is provided a wearable device, comprising: an myoelectric signal acquisition apparatus configured to acquire a surface myoelectric signal; and any of the above motion speed analysis apparatuses.

In some embodiments, the myoelectric signal acquisition apparatus comprises a detector configured to be attached to a surface of a detected user's body.

**BRIEF DESCRIPTION OF THE DRAWINGS**

The accompanying drawings are described herein to provide a further understanding of the present disclosure and constitute a part of the present disclosure. Illustrative embodiments of the present disclosure and the description thereof serve to explain the present disclosure and do not constitute an improper restriction on the present disclosure. In the drawings:

Fig. 1 is a flow diagram of a motion speed analysis method according to some embodiments of the present disclosure.

Fig. 2 is a flow diagram of a motion speed analysis method according to other embodiments of the present disclosure

Fig. 3 is a schematic diagram of threshold adjustment in a motion speed analysis method according to some embodiments of the present disclosure.

Fig. 4 is a schematic diagram of a motion speed analysis apparatus according to some embodiments of the present disclosure.

Fig. 5 is a schematic diagram of a motion speed analysis apparatus according to other embodiments of the present disclosure.

Fig. 6 is a schematic diagram of a motion speed analysis apparatus according to still other embodiments of the present disclosure.

Fig. 7 is a schematic diagram of a wearable device according to some embodiments of the present disclosure.

**DETAILED DESCRIPTION**

Technical solutions of the present disclosure will further described in detail below through the accompanying drawings and embodiments.

Inventors have found that an algorithm of analyzing a motion speed of a user in the related art needs the help of a great number of external auxiliary devices, for example, using a great number of myoelectric channels, or directly measuring an angular speed of a motion portion, both of which need more devices and are inconvenient to carry. In addition, it is difficult to realize motion measurement for an amputated patient due to constraints of muscle positions and limb positions. It is one objective of the present disclosure to expand the application scope of motion speed analysis.

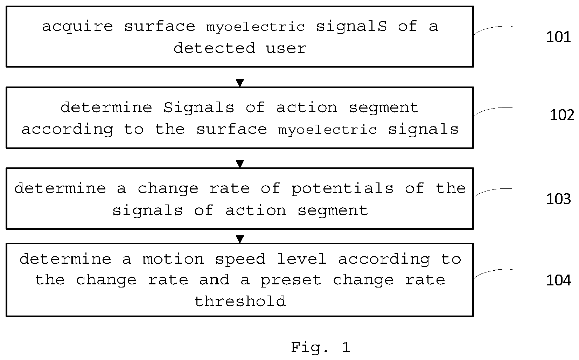

A flow diagram of a motion speed analysis method according to some embodiments of the present disclosure is shown in Fig. 1.

In step 101, surface myoelectric signals of a detected user is acquired. The surface myoelectric signal is a superposition of action potentials of action units of numerous muscle fibers on time and space, which can record and analyze myoelectric signals sent out when a muscle is contracted.

In some embodiments, data may be acquired by using an myoelectric signal acquisition device attached to a surface of the detected user's body, to obtain the surface myoelectric signals. In some embodiments, a directly acquired signal may be taken as an initial surface myoelectric signal, and after a correction process, a surface myoelectric signal is obtained.

In step 102, signals of action segment are determined according to the surface myoelectric signals. In some embodiments, the signals of action segment may be detected by employing methods in the related art, such as a moving average method, standard deviation and absolute mean detection, a wavelet transform method, a statistical criterion decision method, and the like.

In step 103, a change rate of potentials of the signals of action segment is determined. In some embodiments, the change rate of the potentials of the signals of action segment may be determined by calculating an electrical potential difference of the signals of action segment.

In step 104, a motion speed level is determined according to the change rate and a preset change rate threshold. In some embodiments, a motion state may refer to an execution speed of an action of the detected user's portion including limbs, such as an execution speed of a gesture action. Since the surface myoelectric signal is directly related to muscular contraction force, the muscular contraction force is the force of muscle contraction when the limb is in motion at will, and the number of raised active units gradually increases with the increase of the muscular contraction force. That is, the greater the contraction force is, the more active units are raised, so that the degree of superposition between action potential waveforms of the motion units is higher, whose intuitive representation on a waveform of surface myoelectric envelope signal is a higher signal peak value. A change rate of waveforms of surface myoelectric envelope signal of different contraction forces can indirectly characterize the speed, such as the execution speed of the gesture action.

In this way, the motion speed level can be determined according to the surface myoelectric signals generated by muscle contraction mobilized by an organism in order to perform a motion operation, so that the motion speed analysis for people including an amputated patient is realized, which expands the application scope of the motion speed analysis.

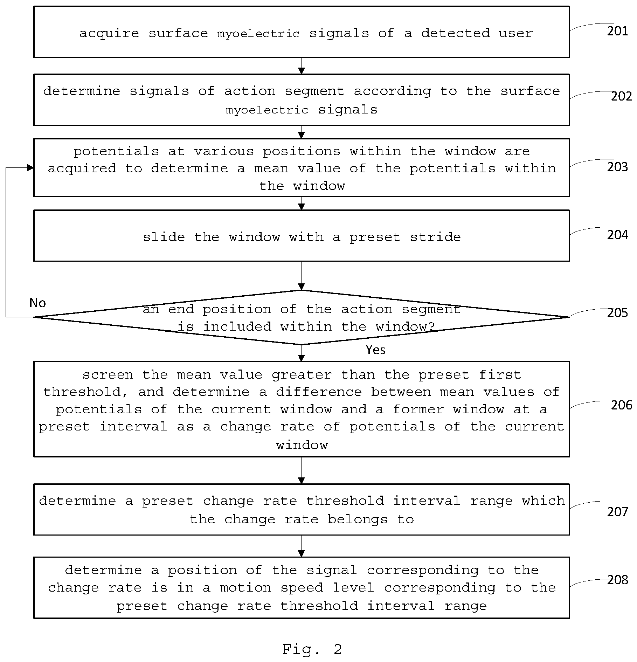

A flow diagram of a motion speed analysis method according to other embodiments of the present disclosure is shown in Fig. 2.

In step 201, surface myoelectric signals of a detected user is acquired. In some embodiments, initial data of the surface myoelectric signals may be acquired by using an myoelectric signal acquisition apparatus attached to a body surface of the detected user, thereby performing a correction operation.

In some embodiments, a baseline threshold may be determined according to data of the surface myoelectric signals in resting-state. In some embodiments, the detected user may be asked to be in a resting state, and data is acquired, which is confirmed resting-state surface myoelectric signal data. In some embodiments, the baseline threshold thr may be determined according to an equation (1) below:

$$\mathtt{thr=mean}\{\mathtt{MAV_1,MAV_2,MAV_3,\cdots,MAV_k}\}\mathtt{+A} \qquad (1)$$

wherein mean is an operator for an averaging operation, $MAV_i$ is a maximum value of the signals within a sliding window in resting-state data of the initial data of surface operator signals, i is a positive integer between 1 and k, k is the number of times that the sliding window slides, and A is a preset constant. In some embodiments, the preset constant A may be set based on experience and adjusted in a testing process to improve accuracy.

The initial data of the surface myoelectric signals is corrected based on the baseline threshold to acquire the surface myoelectric signals. In some embodiments, it may be set that in the case where the initial data of surface myoelectric signal is less than the baseline threshold, the surface myoelectric signal is 0; and in the case where the initial data of the surface myoelectric signal is greater than or equal to the baseline threshold, the surface myoelectric signal is the acquired initial data of surface myoelectric signal. The initial data of the surface myoelectric signal is corrected based on an equation (2) below, to obtain the surface myoelectric signal:

$$s_i = \begin{cases} 0, & x_i < thr; \\ x_i, & x_i > thr; \end{cases} \quad (2)$$

wherein $x_i$ is the acquired initial data of the surface myoelectric signal, and **thr** is the baseline threshold of the initial data of the surface myoelectric signal.

By such a correction, the influence of individual differences can be reduced.

In some embodiments, after the correction is completed, an envelope signal of the corrected signal may be obtained by performing an integral operation on the corrected signal.

In step 202, signals of action segment are determined according to the surface myoelectric signals. There are two kinds of surface myoelectric signals: one is a resting potential, and the other is an action potential which is generated when a muscle is contracted. needs Detecting an starting position of the action potential is needed in determining the signals of action segment. When it is at the resting potential, since initial date of signals have been individually corrected and a waveform amplitude value of the surface myoelectric envelope signal is zero, a position where the amplitude value of the surface myoelectric envelope signal begins to be greater than zero may be regarded as a starting position of the action potential.

In step 203, potentials at various positions within the window are acquired to determine a mean value of the potentials within the window.

In step 204, the window is slid with a preset stride. In some embodiments, a preset value of the width of the window and the preset stride may be set according to a sampling rate, the width of the window does not exceed one quarter of the sampling rate, and the preset stride may be one half of the width of the window.

In step 205, it is determined whether an end position of the action segment is included within the window. If the end position of the action segment is included within the window, then switching to the resting-state, and step 206 is executed; and if the end position of the action segment is not included, the step 203 is executed.

In step 206, when the mean value is greater than a preset first threshold, it is considered that at this moment, a change rate calculated by using the surface myoelectric signals of the muscle is valid, the calculated change rate may characterize a speed of the action , and therefore, the mean value greater than the preset first threshold is screened as a change rate of the potential at a corresponding position, and a difference between mean values of potentials of two windows at a preset interval is taken as a change rate of potentials of a latter window of the two windows. For example: an mean value of the amplitude in the window is **$aver_i$**, if **$aver_i$** $>$ **threshold1**, then:

a change rate of a window i = **$aver_i$** - **$aver_{i-2}$**, where **threshold1** is the preset first threshold, i is a window serial number and is a positive integer, i is greater than 2, and the preset interval is two windows.

In some embodiments, the order of steps 205 and 206 may be exchanged, thereby increasing the efficiency of obtaining the change rate of the various positions in the window.

In step 207, a preset change rate threshold interval range which the change rate belongs to is determined. For example, a preset change rate threshold is set, and it is determined whether the change rate is less than or equal to the preset change rate threshold, or greater than the preset change rate threshold. In other embodiments, n different preset change rate thresholds may be set, thereby dividing the motion speed level into n+1 levels, wherein n is greater than 1.

In step 208, a position of the signal corresponding to the change rate is determined as a motion speed level corresponding to the preset change rate threshold interval range.

For example, in the case that motion speed levels includes two levels:

$$\begin{cases} change\ rate > threshold2, & fast\ speed \\ change\ rate \leq threshold2, & slow\ speed \end{cases} \quad (3)$$

a user may define the preset change rate threshold **threshold2** by himself according to a previous autonomous practice and learn a gesture action's speed to which he can adapt.

In some embodiments, the step 205 executed after the step 208, thereby determining the motion speed level at the current window's position in real time in the moving process of the window, and increasing the running reaction velocity.

In this way, under the condition of no acceleration, no joint angle and no other external auxiliary measurement, the speed of the gesture action can be calculated according to an autonomous control of muscles, and the application scope of the motion speed analysis can be expanded. For an amputated patient, the speed of gesture action can be determined, thereby controlling a bionic hand to execute the gesture action with a corresponding speed, which has great significance for controlling the speed of the action executed by the bionic artificial limb.

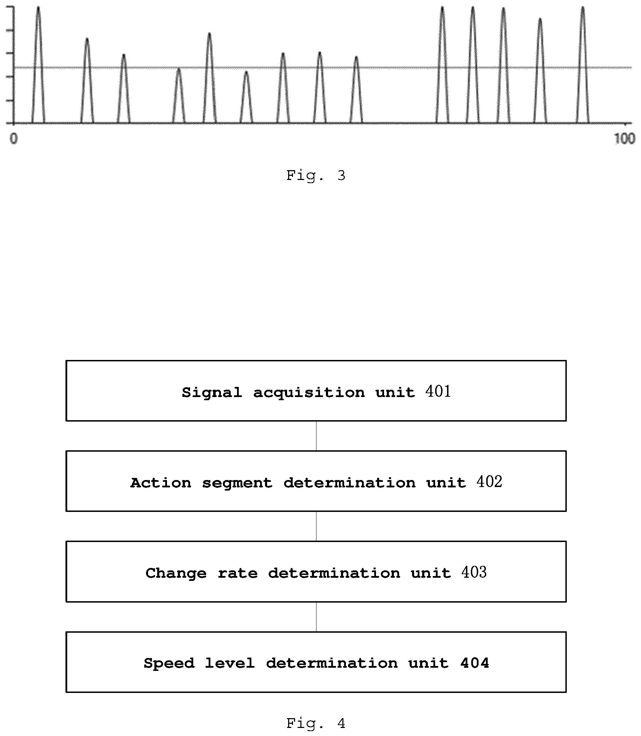

In some embodiments, the preset change rate threshold may be obtained through previous device training, such that the process of the motion analysis conforms to individual characteristics of the users. In some embodiments, after the detected user correctly wears a myoelectric acquisition device, the detected user is first asked to practice a certain gesture action, such as turning outwards, and self-adaptively adjust the execution speed of the gesture action. As shown in Fig. 3, a high peak is a change rate obtained from the recorded surface myoelectric signals according to the above method when the gesture action is fast, and a low peak is a change rate obtained when the gesture action is slow. The height of a horizontal line is self-adaptively adjusted according to an instructed operation performed by the user and the obtained change rate data when the detected user is practicing, and the preset change rate threshold for the gesture action of the user is determined.

In this way, the preset change rate threshold can be made to conform to individual characteristics of the users through the operations of try-on and self-adaptive adjustment, and the degree of individuation and the accuracy of the apparatus are improved.

A schematic diagram of a motion speed analysis apparatus according to some embodiments of the present disclosure is shown in Fig. 4.

A signal acquisition unit 401 is capable of acquiring surface myoelectric signals of a detected user. In some embodiments, the surface myoelectric signals may be acquired by using a myoelectric signal acquisition apparatus attached to a body surface of the detected user. In some embodiments, a directly acquired signal may be taken as an initial surface myoelectric signal, and after a correction process, a surface myoelectric signal is obtained.

An action segment determination unit 402 is capable of determining signals of action segment according to the surface myoelectric signals. In some embodiments, the signals of action segment may be detected by employing methods in the related art, such as a moving average method, standard deviation and absolute mean detection, a wavelet transform method, a statistical criterion decision method, and the like.

A change rate determination unit 403 is capable of determining a change rate of potentials of the signals of action segment. In some embodiments, the change rate of the potentials of the signals of action segment may be determined by calculating an electrical potential difference of the signals of action segment.

A speed level determination unit 404 is capable of determining a motion speed level based on the change rate and a preset change rate threshold.

Such an apparatus can determine the motion speed level according to the surface myoelectric signals generated by muscle contraction mobilized by an organism in order to perform a motion operation, thereby realizing the motion speed analysis of the organism including an amputated patient, thus expanding the application scope of the apparatus.

In some embodiments, the signal acquisition unit 401 is capable of acquiring the initial data of surface myoelectric signal by using a myoelectric signal acquisition apparatus attached to the body surface of the detected user, and then performs a correction operation. In some embodiments, a baseline threshold may be determined according to data of the surface myoelectric signals in resting-state. The initial data of the surface myoelectric signals is corrected based on the baseline threshold to acquire the surface myoelectric signals. In some embodiments, it may be set that in the case where the initial data of surface myoelectric signal is less than the baseline threshold, the surface myoelectric signal is 0; and in the case where the initial data of the surface myoelectric signal is greater than or equal to the baseline threshold, the surface myoelectric signal is the acquired initial data of surface myoelectric signal. By such a correction, the influence of individual differences can be reduced, and the accuracy of the motion analysis can be improved.

In some embodiments, the change rate determination unit 403 is capable of acquiring potentials at various positions within a window, determining a mean value of the potentials within the window, and sliding the window with a preset stride. In some embodiments, a preset value of the width of the window and the preset stride may be set according to a sampling rate, the width of the window does not exceed one quarter of the sampling rate, and the preset stride may be one half of the width of the window.

When the mean value is greater than a preset first threshold, it is considered that at this moment, the change rate calculated by using the surface myoelectric signal of the muscle is valid, and the calculated change rate may characterize the action's speed, and therefore, the mean value greater than the preset first threshold is screened as a change rate of the potential at a corresponding position, and a difference between the mean values of potentials of two windows at a preset interval is taken as a change rate of potentials of a latter window of the two windows.

Such a motion speed analysis apparatus can obtain the change rate of the current position with the acquisition of the surface myoelectric signal and the sliding of the window, so that the running efficiency is ensured; and by ignoring some unreasonable data in the operation process, the data which needs to be operated subsequently can be reduced, so that the operation efficiency is further improved.

In some embodiments, the speed level determination unit 404 is capable of determining a preset change rate threshold interval range which the change rate belongs to, and determining that a position of the signal corresponding to the change rate is in a motion speed level corresponding to the preset change rate threshold interval range. For example, a preset change rate threshold is set, and it is determined whether the change rate is be less than or equal to the preset change rate threshold, or greater than the preset change rate threshold. In other embodiments, n different preset change rate thresholds may be set, thereby dividing the motion speed level into n+1 levels, wherein n is greater than 1.

Such a motion speed analysis apparatus can realize the calculation of the velocity of the gesture action according to autonomous control of the muscle under the condition of no acceleration, joint angle and other external auxiliary measurement, which has great significance for controlling the execution action's speed of a bionic artificial limb; and more refined motion speed analysis can be realized by setting a plurality of motion speed levels, so that the accuracy of the analysis is improved.

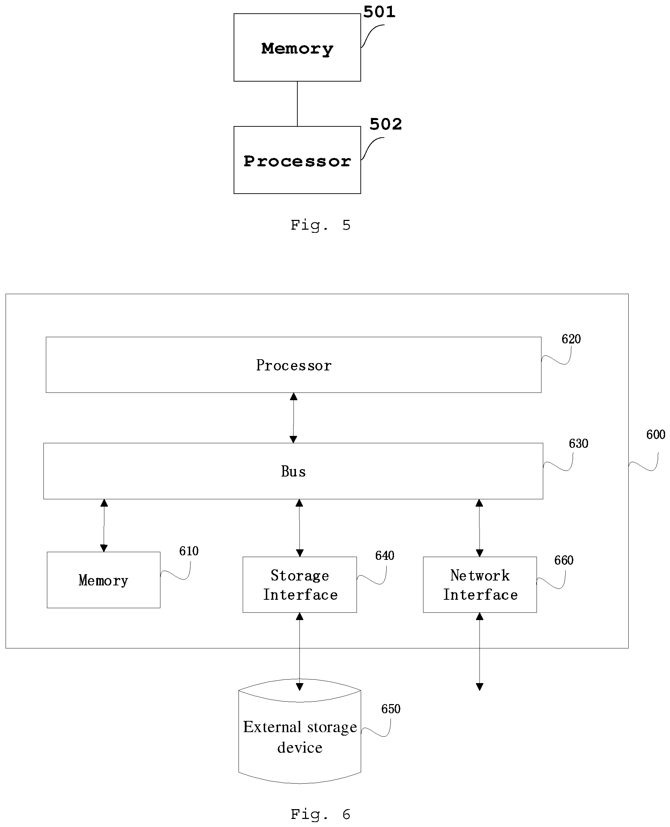

A schematic structural diagram of a motion speed analysis apparatus according to an embodiment of the present disclosure is shown in Fig. 5. The motion speed analysis apparatus comprises a memory 501 and a processor 502. The memory 501 may be a magnetic disk, flash memory, or any other non-volatile storage medium. The memory is configured to store instructions in the embodiments corresponding to the motion speed analysis method above. Coupled to the memory 501 is a processor 502, which may be implemented as one or more integrated circuits, such as a microprocessor or microcontroller. The processor 502 is configured to execute the instructions stored in the memory, so that the motion speed analysis for people including an amputated patient can be realized, and the application scope of the apparatus is expanded.

In one embodiment, as also shown in Fig. 6, the motion speed analysis apparatus 600 comprises a memory 601 and a processor 602. The processor 602 is coupled to the memory 601 through a BUS 603. The motion speed analysis apparatus 600 may also be connected to an external storage apparatus 605 through a storage interface 604 to call external data, and may also be connected to a network or another computer system (not shown) through a network interface 606, which will not be described in detail herein.

In the embodiment, by storing data instructions through the memory and processing the instructions by the processor, the motion speed analysis for one including an amputated patient can be realized, and the application scope of the device is expanded.

In another embodiment, a computer-readable storage medium has stored thereon computer program instructions which, when executed by a processor, implement the steps of the method in the embodiments corresponding to the motion speed analysis method. As will be appreciated by those skilled in the art, the embodiments of the present disclosure may be provided as a method, apparatus, or computer program product. Accordingly, the present disclosure may take a form of an entire hardware embodiment, an entire software embodiment or an embodiment combining software and hardware aspects. Furthermore, the present disclosure may take a form of a computer program product implemented on one or more computer-usable non-transitory storage media (including, but not limited to, disk storage, CD-ROM, optical storage, and the like) having computer-usable program code embodied therein.

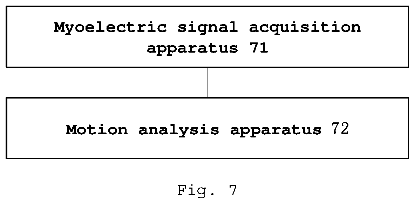

A schematic diagram of a wearable device according to some embodiments of the present disclosure is shown in Fig. 7. An myoelectric signal acquisition apparatus 71 may be a detector attached to a surface of a detected user's body, capable of acquiring a user's surface myoelectric signal. In some embodiments, the myoelectric signal acquisition apparatus 71 detects a portion of the user's body, such as an arm, and then analyzes an execution speed of the user's gesture action. A motion speed analysis apparatus 72 may be any of those mentioned above. The motion speed analysis apparatus 72 may be integrated in a terminal of the wearable device, or detection data may be transmitted by the detector to a remote data processing side in a wired or wireless manner, and the motion speed analysis apparatus 72 on the data processing side performs the motion speed analysis method as mentioned above.

Such a wearable device can determine the motion speed level according to the surface myoelectric signals generated by muscle contraction mobilized by an organism in order to perform a motion operation, so that the motion speed analysis for people including an amputated patient is realized, which expands the application scope of the device.

In some embodiments, the wearable device may also be a bionic artificial limb in addition to a somatosensory interaction device, and by determining a gesture action speed of an amputated user and thereby controlling the bionic hand to execute a gesture action with a corresponding speed, the living standard of the amputated patient is improved.

The present disclosure is described with reference to flow diagrams and/or block diagrams of the method, device (system) and computer program product according to the embodiments of the disclosure. It should be understood that each flow and/or block in the flow diagrams and/or block diagrams, and combinations of flows and/or blocks in the flow diagrams and/or block diagrams, can be implemented by computer program instructions. These computer program instructions may be provided to a processor of a general-purpose computer, special-purpose computer, embedded processor, or other programmable data processing device to produce a machine, such that the instructions, which are executed by the processor of the computer or other programmable data processing device, create means for implementing functions specified in a flow or flows of the flow diagrams and/or a block or blocks of the block diagrams.

These computer program instructions may also be stored in a computer-readable memory that can guide a computer or other programmable data processing device to work in a specific manner, such that the instructions stored in the computer-readable memory produce an article of manufacture including an instruction device which implements functions specified in a flow or flows of the flow diagrams and/or a block or blocks of the block diagrams.

These computer program instructions may also be loaded onto a computer or other programmable data processing device to cause a series of operation steps to be performed on the computer or other programmable device to produce a computer-implemented process, such that the instructions executed on the computer or other programmable device provide steps for implementing functions specified in a flow or flows of the flow diagrams and/or a block or blocks of the block diagrams.

Thus far, the present disclosure has been described in detail. Some details well known in the art have not been described in order to avoid obscuring the concepts of the present disclosure. Those skilled in the art may fully appreciate how to implement the technical solutions disclosed herein, in view of the foregoing description.

The method and apparatus of the present disclosure may be implemented in a number of ways. For example, the method and apparatus of the present disclosure may be implemented by software, hardware, firmware, or any combination of software, hardware, and firmware. The above-described order for the steps of the method is for illustration only, and the steps of the method of the present disclosure are not limited to the order specifically described above unless specifically stated otherwise. Further, in some embodiments, the present disclosure may also be implemented as programs recorded in a recording medium, and these programs include machine-readable instructions for implementing the method according to the present disclosure. Therefore, the present disclosure further covers the recording medium having stored thereon the programs for executing the method according to the present disclosure.

Finally, it should be noted that: the above embodiments are intended only to illustrate the technical solutions of the present disclosure and not to limit them; and although the present disclosure has been described in detail with reference to the preferred embodiments, those of ordinary skill in the art should understand that: modifications to the specific implementations of the present disclosure or equivalent substitutions for parts of the technical features may be made, all of which are intended to be covered within the scope of the technical solutions claimed in this disclosure without departing from the spirit thereof.

**Claims**

1. A motion speed analysis method, comprising:

    acquiring surface myoelectric signals of a detected user;
    determining signals of action segment according to the surface myoelectric signals;
    determining a change rate of potentials of the signals of action segment; and
    determining a motion speed level according to the change rate and a preset change rate threshold.

2. The motion speed analysis method according to claim 1, wherein determining the change rate of potentials of the signals of action segment comprises:

    determining a mean value of the potentials within a window from an initial position of the signals of action segment;
    sliding the window with a preset stride, and obtaining a mean value of the potentials within the window after each sliding; and
    taking a difference between mean values of the potentials of two windows spaced at a preset interval as a change rate of the potentials of a latter window of the two windows.

3. The motion speed analysis method according to claim 2, wherein determining the change rate of the potentials of the signals of action segment further comprises:

    comparing a mean value of the potentials of a current window with a preset first threshold;
    if the mean value of the potentials of the current window is greater than the preset first threshold, determining a difference between the mean value of the potentials of the current window and a mean value of the potentials of a former window with the preset interval from the current window, and taking the difference as a change rate of the potentials of the current window; and
    if the mean value of the potentials of the current window is less than or equal to the preset first threshold, ignoring the change rate of the potentials of the current window.

4. The motion speed analysis method according to claim 1, 2, or 3, wherein determining the motion speed level according to the change rate and the preset change rate threshold comprises:

comparing the change rate with the preset change rate threshold;

if the change rate is less than or equal to the preset change rate threshold, determining that a motion state of the user is a first motion speed level; and

if the change rate is greater than the preset change rate threshold, determining that the motion state of the user is a second motion speed level,

wherein a speed at the second motion speed level is larger than the speed at the first motion speed level.

5. The motion speed analysis method according to claim 1, 2, or 3, wherein determining the motion speed level according to the change rate and the preset change rate threshold comprises:

determining a preset change rate threshold interval range where the change rate belongs to; and

determining that at a position of a signal to which the change rate corresponds, a motion state of the user is a motion speed level to which the preset change rate threshold interval range corresponds,

wherein the number of motion speed levels is greater than 2.

6. The motion speed analysis method according to claim 1, further comprising:

respectively acquiring data of the surface myoelectric signals of the user in various motion speed states; and

determining the preset change rate threshold according to the data of the surface myoelectric signals.

7. The motion speed analysis method according to claim 1, wherein acquiring the surface myoelectric signal of the detected user comprises:

acquiring initial data of surface myoelectric signals; and

acquiring the surface myoelectric signals by correcting the initial data of surface myoelectric signals based on a baseline threshold.

8. The motion speed analysis method according to claim 7, wherein acquiring the surface myoelectric signals of the detected user further comprises:

determining the baseline threshold thr according to an equation:

$$\mathrm{thr=mean}\{MAV_1, MAV_2, MAV_3, ..., MAV_k\}+A$$

wherein MAVi is a maximum value of the signals within a sliding window in resting-state data of the initial data of the surface myoelectric signals, i is a positive integer between 1 and k, k is the number of times that the sliding window slides, and A is a preset constant.

9. A motion speed analysis apparatus, comprising:

a signal acquisition unit configured to acquire surface myoelectric signals of a detected user;

an action segment determination unit configured to determine signals of action segment according to the surface myoelectric signals;

a change rate determination unit configured to determine a change rate of potentials of the signals of action segment; and

a speed level determination unit configured to determine a motion speed level according to the change rate and a preset change rate threshold.

10. The motion speed analysis apparatus according to claim 9, wherein the change rate determination unit is configured to:

determine a mean value of the potentials within a window from an initial position of the signals of action segment;

slide the window with a preset stride, and obtaining a mean value of the potentials within the window after each sliding; and

take a difference between mean values of the potentials of two windows spaced at a preset interval as a change rate of the potentials of a latter window of the two windows.

11. The motion speed analysis apparatus according to claim 9 or 10, wherein the speed level determination unit is configured to:

> determine a preset change rate threshold interval range where the change rate belongs to; and
> determine that at a position of a signal to which the change rate corresponds, a motion state of the user is a motion speed level to which the preset change rate threshold interval range corresponds;
> wherein the number of motion speed levels is greater than 2.

12. A motion speed analysis apparatus, comprising:

> a memory; and
> a processor coupled to the memory, wherein the processor is configured to, based on instructions stored in the memory, perform the method according to any of claims 1 to 8.

13. A computer-readable storage medium having stored thereon computer program instructions, which when executed by a processor, implement steps of the method according to any of claims 1 to 8.

14. A wearable device, comprising:

> an myoelectric signal acquisition apparatus configured to acquire a surface myoelectric signal; and
> the motion speed analysis apparatus according to any of claims 9 to 12.

15. The wearable device according to claim 14, wherein the myoelectric signal acquisition apparatus comprises a detector configured to be attached to a surface of a detected user's body.

```
┌────────────────────────────────────────┐
│    acquire surface myoelectric signalS of a │          101
│               detected user              │
└────────────────────────────────────────┘
                    │
                    ▼
┌────────────────────────────────────────┐
│    determine Signals of action segment   │          102
│  according to the surface myoelectric signals │
└────────────────────────────────────────┘
                    │
                    ▼
┌────────────────────────────────────────┐
│ determine a change rate of potentials of the │       103
│         signals of action segment        │
└────────────────────────────────────────┘
                    │
                    ▼
┌────────────────────────────────────────┐
│ determine a motion speed level according to │        104
│   the change rate and a preset change rate │
│                threshold                 │
└────────────────────────────────────────┘
```

Fig. 1

acquire surface myoelectric signals of a detected user — 201

determine signals of action segment according to the surface myoelectric signals — 202

potentials at various positions within the window are acquired to determine a mean value of the potentials within the window — 203

slide the window with a preset stride — 204

an end position of the action segment is included within the window? — 205

No

Yes

screen the mean value greater than the preset first threshold, and determine a difference between mean values of potentials of the current window and a former window at a preset interval as a change rate of potentials of the current window — 206

determine a preset change rate threshold interval range which the change rate belongs to — 207

determine a position of the signal corresponding to the change rate is in a motion speed level corresponding to the preset change rate threshold interval range — 208

Fig. 2

Fig. 3

| |
|---|
| Signal acquisition unit 401 |
| Action segment determination unit 402 |
| Change rate determination unit 403 |
| Speed level determination unit 404 |

Fig. 4

Fig. 5

Fig. 6

```
┌──────────────────────────────────────────────┐
│        Myoelectric signal acquisition         │
│                apparatus 71                    │
└──────────────────────────────────────────────┘
                        │
┌──────────────────────────────────────────────┐
│         Motion analysis apparatus 72          │
└──────────────────────────────────────────────┘
```

Fig. 7

EP 4 005 473 A1

## INTERNATIONAL SEARCH REPORT

| International application No. |
| --- |
| **PCT/CN2020/109444** |

**A. CLASSIFICATION OF SUBJECT MATTER**

A61B 5/11(2006.01)i; A61B 5/0488(2006.01)i; A61F 2/72(2006.01)i

According to International Patent Classification (IPC) or to both national classification and IPC

**B. FIELDS SEARCHED**

Minimum documentation searched (classification system followed by classification symbols)

A61B,A61F

Documentation searched other than minimum documentation to the extent that such documents are included in the fields searched

Electronic data base consulted during the international search (name of data base and, where practicable, search terms used)

CNKI, CNPAT, WPI, EPODOC: 北京海益同展信息科技有限公司, 田彦秀, 韩久琦, 牛天增, 表面, 肌电, 动作, 动作段, 行动, 速度, 电位, 电势, 电压, 变化, 差, surface, electromyography, EMG, act+, motion, speed, velocity, potential, voltage, pressure, change, mutation, variable, rate, differen+, value

**C. DOCUMENTS CONSIDERED TO BE RELEVANT**

| Category* | Citation of document, with indication, where appropriate, of the relevant passages | Relevant to claim No. |
| --- | --- | --- |
| PX | CN 110403609 A (BEIJING HAIYI TONGZHAN INFORMATION TECHNOLOGY CO., LTD.) 05 November 2019 (2019-11-05) claims 1-14, description paragraphs [0007]-[0091], figures 1-7 | 1-15 |
| A | CN 104127181 A (HANGZHOU DIANZI UNIVERSITY) 05 November 2014 (2014-11-05) description, paragraphs [0065]-[0073], and figures 1-4 | 1-15 |
| A | CN 108309294 A (SHANGHAI UNIVERSITY OF MEDICINE & HEALTH SCIENCES) 24 July 2018 (2018-07-24) entire document | 1-15 |
| A | CN 107961135 A (SEIKO EPSON CORPORATION) 27 April 2018 (2018-04-27) entire document | 1-15 |
| A | CN 106156524 A (NORTHEASTERN UNIVERSITY) 23 November 2016 (2016-11-23) entire document | 1-15 |
| A | CN 104873187 A (OYMOTION TECHNOLOGIES, INC.) 02 September 2015 (2015-09-02) entire document | 1-15 |
| A | CN 108968952 A (YANSHAN UNIVERSITY) 11 December 2018 (2018-12-11) entire document | 1-15 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **26 October 2020** | **19 November 2020** |

| Name and mailing address of the ISA/CN | Authorized officer |
| --- | --- |
| **China National Intellectual Property Administration (ISA/CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

16

## INTERNATIONAL SEARCH REPORT

International application No.

**PCT/CN2020/109444**

### C. DOCUMENTS CONSIDERED TO BE RELEVANT

| Category* | Citation of document, with indication, where appropriate, of the relevant passages | Relevant to claim No. |
|---|---|---|
| A | JP 2015211705 A (JAPAN HEALTH SCIENCE FOUND) 26 November 2015 (2015-11-26) entire document | 1-15 |
| A | US 2017312576 A1 (NATARAJAN, Senthil et al.) 02 November 2017 (2017-11-02) entire document | 1-15 |

Form PCT/ISA/210 (second sheet) (January 2015)

INTERNATIONAL SEARCH REPORT
Information on patent family members

International application No.

**PCT/CN2020/109444**

| Patent document cited in search report | | | Publication date (day/month/year) | Patent family member(s) | | | Publication date (day/month/year) |
|---|---|---|---|---|---|---|---|
| CN | 110403609 | A | 05 November 2019 | CN | 110403609 | B | 01 September 2020 |
| CN | 104127181 | A | 05 November 2014 | CN | 104127181 | B | 12 April 2017 |
| CN | 108309294 | A | 24 July 2018 | | None | | |
| CN | 107961135 | A | 27 April 2018 | US | 2018108271 | A1 | 19 April 2018 |
| | | | | JP | 2018064740 | A | 26 April 2018 |
| CN | 106156524 | A | 23 November 2016 | CN | 106156524 | B | 28 August 2018 |
| CN | 104873187 | A | 02 September 2015 | | None | | |
| CN | 108968952 | A | 11 December 2018 | | None | | |
| JP | 2015211705 | A | 26 November 2015 | JP | 6340528 | B2 | 13 June 2018 |
| US | 2017312576 | A1 | 02 November 2017 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910827590